(19) Europäisches Patentamt
European Patent Office
Office européen des brevets

(11) Numéro de publication : **0 586 267 A1**

(12) # DEMANDE DE BREVET EUROPEEN

(21) Numéro de dépôt : **93401733.6**

(51) Int. Cl.$^5$ : **A61L 25/00**

(22) Date de dépôt : **05.07.93**

(30) Priorité : **06.07.92 FR 9208304**

(43) Date de publication de la demande : **09.03.94 Bulletin 94/10**

(84) Etats contractants désignés : **AT BE CH DE DK ES GB IT LI NL PT SE**

(71) Demandeur : **Laboratoires Merck-Clévenot
5-9 Rue Anquetil
F-94130 Nogent-sur-Marne (FR)**

(72) Inventeur : **Villenave, Jean-Jacques
12 allée de Lanquais
F-33170 Gradignan (FR)**
Inventeur : **Alcorta, José Ramon
83 rue Léo Saignat
F-33000 Bordeaux (FR)**

(74) Mandataire : **Orès, Bernard et al
Cabinet ORES 6, Avenue de Messine
F-75008 Paris (FR)**

(54) **Pâte adhésive thixotrope pour la protection de la peau, son procédé de préparation et ses applications.**

(57) Pâte adhésive thixotrope pour la protection de la peau, son procédé de préparation, ses applications thérapeutiques, notamment son utilisation en association avec des appareillages de drainage ou de stomie, et compositions pharmaceutiques la contenant.

Ladite pâte adhésive, comprenant au moins un agent filmogène, une charge hydrophile et un solvant alcoolique, est caractérisée en ce que l'agent filmogène est choisi parmi des copolymères de styrène et d'anhydride maléique solubles dans l'alcool, la charge hydrophile est une association de polyvinylpyrrolidone et d'au moins un hydrocolloïde soluble et/ou gonflant dans l'eau, laquelle pâte adhésive contient, en outre, au moins un agent thixotropant.

EP 0 586 267 A1

La présente invention est relative à une pâte adhésive thixotrope pour la protection de la peau, à son procédé de préparation, à ses applications thérapeutiques, notamment son utilisation en association avec des appareillages de drainage ou de stomie, et aux compositions pharmaceutiques la contenant.

Chez les patients porteurs de tels appareillages, il existe des difficultés à maintenir une étanchéité correcte entre ces appareillages et la peau qui entoure l'orifice chirurgical ménagé à la surface du corps (site de drainage ou stomie). Le problème des fuites de liquide est aggravé lorsque la peau entourant cet orifice est irrégulière ou lorsqu'elle peut se plisser. Dans de telles conditions, même si le dispositif de recueil comporte un élément adhésif de scellement souple (anneau, pansement...), une étanchéité totale aux liquides organiques ne peut être garantie. Pour obtenir un joint de meilleure qualité, il convient de disposer de revêtements adhésifs se présentant sous forme de pâtes, qui sont appliquées sur la peau et séchées avant la mise en place de l'appareillage.

Les qualités demandées à ces pâtes sont donc, en premier lieu, une bonne adhésivité, y compris sur des surfaces cutanées humides, une souplesse d'application leur permettant de combler les irrégularités de la peau et une excellente biocompatibilité, pour ne pas produire d'irritation sur une peau déjà fragilisée.

Dans certains sites de drainage ou de stomie, la peau entourant le site est exposée à l'action corrosive des effluents de pH variable : sucs intestinaux, bile, urine... Ainsi, une autre caractéristique des pâtes adhésives protectrices doit être une résistance convenable à l'action de ces effluents. Cette résistance peut, seule, assurer le maintien à un niveau satisfaisant des propriétés mécaniques, adhésives et protectrices, pendant des durées suffisantes pour apporter un bon confort d'utilisation aux patients.

De manière générale, les pâtes adhésives décrites dans l'Art antérieur comprennent au moins un agent filmogène leur donnant leur élasticité et leur adhésivité, au moins un agent hydrophile, gonflant dans l'eau, destiné à conserver l'adhésivité sur peau humide ainsi qu'un solvant, permettant, par évaporation, de constituer rapidement une pellicule à la surface de la peau.

Le Brevet HOLLISTER FR 2 467 873 et le Brevet US 4 350 785 décrivent une pâte adhésive protectrice pour appareillage de stomie, qui comprend un mélange d'hydrocolloïdes en poudre absorbant l'eau, une résine filmogène adhésive choisie parmi les hémi-esters d'alkyle de copolymères alternés de l'éther de méthyle et de vinyle et de l'anhydride maléique [poly(EMV-*co*-AM)], en solution alcoolique et 2 à 6 % de silice colloïdale.

Le Brevet européen SQUIBB 48 556 décrit une pâte adhésive pour la protection et le traitement de la peau autour d'une stomie, qui comprend des hydrocolloïdes, une solution alcoolique de l'hémi-ester butylique partiel d'un poly(EMV-*co*-AM), en tant que résine filmogène, des agents gélifiants et épaississants, un plastifiant et d'autres agents, si nécessaire.

Cependant, les pâte protectrices proposées conformément à l'Art antérieur ne présentent pas toutes les qualités requises et notamment celle d'un maintien de l'adhésivité à long terme. En effet, l'un des facteurs d'irritation de la peau, liés à l'utilisation d'appareillages de drainage ou de stomie, est le "trauma d'adhésion", induit par l'application et l'enlèvement répétés de ces appareillages. Ceci est particulièrement vrai pendant la période post-opératoire, période pendant laquelle la peau entourant la stomie est traumatisée, ulcérée, inflammatoire. Il est donc souhaitable sur le plan médical, mais aussi sur un plan économique, de disposer d'une pâte capable de conserver son adhésivité et son étanchéité pendant plusieurs jours, voire une semaine, tout en pouvant être enlevée facilement, si nécessaire.

En conséquence, la Demanderesse a cherché à résoudre le problème de l'obtention d'une pâte adhésive pour la protection de la peau, qui présente effectivement toutes les propriétés requises pour de telles applications, à savoir :

. adhérer à la peau par simple étalement ;
. sécher rapidement après son application pour former un film protecteur, tout en étant ni toxique, ni irritante pour la peau,
. avoir une cohésion importante, la rendant utilisable en couches plus ou moins épaisses (obtention d'une surface plane et lisse, rattrapage des différences de dimensions entre les anneaux adhésifs des appareillages et les orifices de drainage ou de stomie) ;
. assurer l'adhésion et l'étanchéité immédiate de l'appareillage destiné à être mis en place ;
. avoir des propriétés plastiques et élastiques, pour épouser en permanence la forme de la peau, tout en ne subissant pas de fluage notable sous charge ;
. absorber l'humidité de la peau (sueur) ainsi que les excrétions corporelles (sucs intestinaux, urine, ...), tout en présentant une résistance convenable à la dégradation par ces fluides ;
. être utilisable dans tous les cas qui nécessitent une protection cutanée : appareillages de drainage, de stomie, de fistules, d'incontinence, support de pansements et d'adhésifs, support de tout dispositif médical ;
. être compatible avec les différents matériaux utilisés en médecine ou chirurgie (films, sondes de drainage, cathéters, poches de drainage...) ;

. être formulée à partir d'un nombre limité de constituants, de manière à assurer la simplification de la fabrication et des prix de revient acceptables ;
. être stable à la conservation (au moins 5 ans dans des conditions normales de stockage) ; et surtout
. adhérer à la peau et maintenir l'étanchéité requise, pendant un temps suffisamment long (de 2 à 7 jours).

La présente invention s'est en conséquence donné pour but de pourvoir à une pâte pour la protection de la peau, présentant tous les avantages énoncés plus haut.

C'est encore un but de l'invention de pourvoir à un procédé de préparation de ladite pâte protectrice.

C'est, en outre, un but de l'invention de pourvoir à des compositions pharmaceutiques la contenant.

La présente invention a pour objet une pâte adhésive pour la protection de la peau, comprenant au moins un agent filmogène, une charge hydrophile et un solvant alcoolique, caractérisée en ce que l'agent filmogène est choisi parmi des copolymères de styrène et d'anhydride maléique solubles dans l'alcool, la charge hydrophile est une association de polyvinylpyrrolidone et d'au moins un hydrocolloïde soluble et/ou gonflant dans l'eau, et en ce qu'elle contient, en outre, au moins un agent thixotropant.

Selon un mode de réalisation avantageux de l'invention, ladite pâte comprend, en outre, au moins l'un des additifs suivants : un diluant huileux et/ou un agent odoriférant et/ou une résine tackifiante.

De manière avantageuse, l'agent filmogène est choisi parmi les copolymères de styrène et d'anhydride maléique partiellement estérifiés par des alcools et présentant la formule I ci-après :

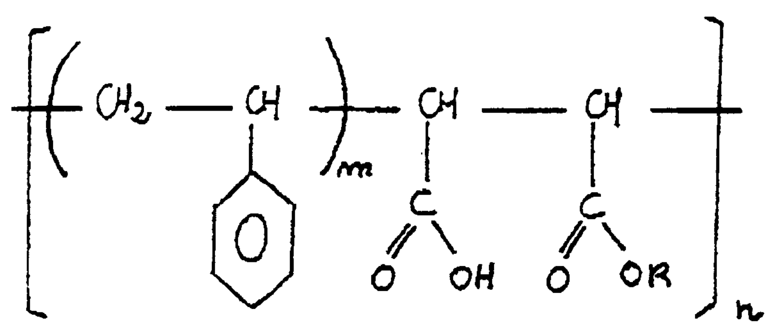

dans laquelle m = 1-3 et n = 6-8.

Ces copolymères sont commercialisés sous le nom de résines SMA® par la Société ATOCHEM INC., notamment pour la fabrication d'encres, de papiers, de peintures, de revêtements thermodurcissables, de cires, de shampooings pour sols, de dispersants pour l'agriculture ou le collage du papier. Aussi, c'est de manière inattendue que la Demanderesse a constaté qu'ils présentent une biocompatibilité, permettant de les utiliser comme agent filmogène dans la présente invention.

De manière particulièrement avantageuse, l'agent filmogène est l'hémi-ester du butylcellosolve du copolymère de styrène et d'anhydride maléique, référencé SMA® 1440-A. Ce dernier est, parmi les formes estérifiées de ces résines, celle ayant la masse molaire la plus basse (2500) et le rapport styrène/anhydride maléique le plus faible (1:1).

En raison de la présence des motifs styrène, les copolymères de styrène et d'anhydride maléique sont des composés de nature hydrophobe. Leurs formes partiellement estérifiées sont solubles dans l'éthanol et les solvants alcooliques, mais insolubles dans l'eau.

La polyvinylpyrrolidone utile selon l'invention peut être choisie dans la gamme des masses molaires comprises entre 10 000 et 40 000. Elle se présente sous forme d'une poudre très fine, soluble dans les solvants alcooliques et pouvant être intimement mélangée au copolymère de styrène et d'anhydride maléique en présence d'alcool. Elle constitue, de par ses propres propriétés filmogènes, sa pégosité et son hydrophilie un complément parfait du copolymère de styrène et d'anhydride maléique, le résultat étant une adhésivité de la pâte obtenue sur peau humide nettement supérieure à celle des pâtes de l'Art antérieur.

Selon un autre mode de réalisation avantageux de l'invention, le rapport en masses agent filmogène:polyvinylpyrrolidone est compris entre 3,5:1 et 2:1.

Conformément à l'invention, le solvant est notamment choisi parmi l'éthanol, le n-propanol, l'isopropanol ou un mélange de ceux-ci.

L'éthanol est utilisé de préférence car, en raison de sa grande volatilité, il permet à la pâte de former rapidement un film après son application sur la peau.

La pâte adhésive selon l'invention comprend un ou plusieurs hydrocolloïdes ; ces hydrocolloldes sont habituellement utilisés dans des formulations adhésives à usage cutané, dans le but que ces dernières conservent leurs propriétés adhésives, en présence d'humidité.

Les hydrocolloïdes utiles selon l'invention sont choisis parmi :

- les polysaccharides : carboxyméthylcelluloses (sodiques), hydroxypropylcelluloses, amidons, pectines, dextranomères, gommes naturelles (arabique, de guar, de caroube, de karaya, adragante, xanthane), alginates,...
- les polypeptides : gélatine, caséine, gluten,...,
- les polymères de synthèse hydrosolubles tels que les polyoxyéthylènes ou les polyacrylamides.

Conformément à l'invention, un mélange préféré d'hydrocolloïdes comprend de la gélatine, une pectine, une carboxyméthylcellulose sodique (CMC-Na) et une hydroxypropylcellulose (HPC). Ces composés se présentent sous forme de poudres fines, sèches au toucher et ne comprennent pas plus de 15 % en masse d'humidité.

La pâte selon l'invention est dite thixotrope, c'est-à-dire qu'elle présente une viscosité élevée en l'absence de contraintes de cisaillement et s'abaissant rapidement lorsque la vitesse de cisaillement augmente. Ainsi, la pâte peut être facilement sortie de son conditionnement tel qu'un tube par exemple, puis être étalée sur la peau par simple pression du doigt, tout en ne coulant pas lors de son application.

L'agent thixotropant utile selon l'invention est, de préference, choisi parmi les dérivés solides de l'huile de ricin (tels que le THIXOMEN® commercialisé par la Société ICI, se présentant sous forme d'une poudre finement divisée) ou les poudres minérales de la famille des argiles smectites, notamment la bentonite et/ou la montmorillonite (SIMAGEL SI 345® de la Société STEARINERIE DUBOIS FILS).

La pâte adhésive qui comprend, conformément à l'invention, les constituants suivants : résine SMA®, PVP, hydrocolloïdes solubles et/ou gonflant dans l'eau, agent thixotropant, solvant alcoolique, présente un maintien nettement amélioré de l'adhésivité à long terme, plus particulièrement due à l'action synergique résine SMA®/PVP, et est thixotrope.

La pâte adhésive selon l'invention peut, en outre, contenir un diluant huileux qui a pour effet d'abaisser le module élastique de l'agent filmogène de ladite pâte. Des huiles blanches paraffiniques de qualité pharmaceutique telles que celles distribuées par SHELL, sous le nom d'ONDINA®, conviennent particulièrement bien à la présente invention. Mais d'autres diluants huileux tels que les lactates d'acides gras (lactate de myristyle, lactate de cétyle...) ou les adipates (adipate de 2-éthylhexyle par exemple) peuvent également être utilisés.

De manière avantageuse, la pâte adhésive peut également contenir, en outre, un agent odoriférant. De préférence, celui-ci est la vanilline, mais des essences aromatiques telles que le menthol et ses dérivés peuvent également convenir.

Pour augmenter le caractère d'adhésif sensible à la pression de la pâte, celle-ci peut comprendre, en outre, une ou plusieurs résines tackifiantes. Ces dernières sont de préference choisies parmi les résines terpéniques telles que les esters de collophane ou les polypinènes. Une résine particulièrement préferée pour la présente invention est l'ester méthylique de collophane hydrogénée commercialisé sous la marque HYDROGRAL-M® par la Société DRT.

Selon un autre mode de réalisation préférée de ladite pâte, elle comprend en parties en masse :

. 10 à 40 parties d'agent filmogène,
. 5 à 15 parties de polyvinylpyrrolidone,
. 5 à 60 parties d'hydrocolloïdes,
. 1 à 5 parties d'agent thixotropant,
. 10 à 40 parties de solvant alcoolique,
. 0 à 10 parties de diluant huileux,
. 0 à 20 parties de résine tackifiante,
. 0 à 1 partie d'agent odoriférant.

Une formule préférée de la pâte adhésive conforme à l'invention présente la formulation qualitative et quantitative suivante, exprimée en parties en masse :

. 20 à 30 parties de l'hémi-ester du butylcellosolve du copolymère de styrène/anhydride maléique,
. 8 à 12 parties de polyvinylpyrrolidone,
. 24 à 50 parties d'hydrocolloïdes :
dont
  - 8 à 15 parties de gélatine,
  - 8 à 15 parties de pectine,
  - 8 à 15 parties de carboxyméthylcellulose sodique,
  - 0 à 5 parties d'hydroxypropylcellulose,
. 1 à 3 parties d'agent thixotropant,
. 20 à 25 parties d'éthanol,
. 2 à 10 parties de diluant huileux,
. 0 à 10 parties de résine tackifiante,

. 0,1 partie d'agent odoriférant.

Une formule particulièrement préférée de la pâte adhésive conforme à l'invention présente la formulation qualitative et quantitative suivante, exprimée en parties en masse :

. 25 parties de l'hémi-ester du butylcellosolve du copolymère de styrène/anhydride maléique,
. 10 parties de polyvinylpyrrolidone,
. 37 parties d'hydrocolloïdes :
dont
- 12 parties de gélatine,
- 12 parties de pectine,
- 12 parties de carboxyméthylcellulose sodique,
- 1 partie d'hydroxypropylcellulose,

. 1 partie d'agent thixotropant,
. 25 parties d'éthanol,
. 2 parties de diluant huileux,
. 0,1 partie de vanilline.

L'invention a également pour objet un procédé de préparation de ladite pâte adhésive, caractérisé en ce que :

- dans un malaxeur, on mélange l'agent filmogène et le solvant alcoolique pour obtenir une solution alcoolique dudit agent filmogène ; on ajoute à celle-ci la polyvinylpyrrolidone, puis, le cas échéant, le diluant huileux et/ou la résine tackifiante et on homogénéise ce mélange ;
- on y incorpore le ou les hydrocolloïdes, l'agent thixotropant, et, le cas échéant, l'agent odoriférant et l'ensemble est malaxé jusqu'à l'obtention d'une pâte totalement homogène.

L'invention a également pour objet des compositions pharmaceutiques pour la protection de la peau, caractérisées en ce qu'elles comprennent en tant que constituant actif une pâte conforme à l'invention.

L'invention a, de plus, pour objet des compositions pharmaceutiques, caractérisées en ce qu'elles contiennent en combinaison une pâte conforme à l'invention et d'autres substances à activité thérapeutique.

L'invention a, en outre, pour objet des compositions pharmaceutiques à diffusion contrôlée, caractérisées en ce qu'elles comprennent en tant que véhicule de diffusion, la pâte conforme à l'invention.

L'invention sera mieux comprise à l'aide du complément de description qui va suivre, qui se réfère à des exemples de mise en oeuvre du procédé conforme à l'invention et de réalisation de pâtes adhésives pour la protection de la peau conformes à l'invention.

Il doit être bien entendu, toutefois, que ces exemples sont donnés uniquement à titre d'illustration de l'objet de l'invention, dont ils ne constituent en aucune manière une limitation.

**EXEMPLE 1 : PROCEDE DE PREPARATION D'UNE PATE CONFORME A L'INVENTION AVEC EN PARTIES EN MASSE POUR 100 PARTIES DE PATE : SMA® 25 ET PVP 10.**

a) dans un malaxeur à fort cisaillement, on mélange à parts égales en masse du SMA 1440-A® et de l'éthanol pour obtenir une solution alcoolique à 50 % de résine filmogène ;
b) on ajoute, à 50 parties de cette solution de résine filmogène, 10 parties de polyvinylpyrrolidone. On mélange jusqu'à complète homogénéisation ;
c) on ajoute au mélange ainsi obtenu, 2 parties d'huile blanche paraffinique ONDINA-32® et on homogénéise le tout ;
d) on introduit alors lentement dans le malaxeur, un mélange solide pulvérulent contenant 12 parties de gélatine, 12 parties de pectine, 12 parties de carboxyméthylcellulose sodique et 1 partie d'hydroxypropylcellulose, puis 1 partie de THIXOMEN® et une quantité de vanilline égale à 0,1 % de la composition finale. Le malaxage est poursuivi, à faible vitesse, jusqu'à complète homogénéisation.
La pâte obtenue a donc la composition suivante :

| | Parties en masse pour 100 parties de pâte |
|---|---|
| SMA 1440-A® | 25 |
| PVP | 10 |
| Gélatine | 12 |
| Pectine | 12 |
| CMC Na | 12 |
| Hydroxypropylcellulose | 1 |
| THIXOMEN® | 1 |
| Ethanol | 25 |
| Huile paraffinique ONDINA-32® | 2 |
| Vanilline | 0,1 %. |

**EXEMPLE 2 : PROCEDE DE PREPARATION D'UNE PATE CONFORME A L' INVENTION AVEC EN PARTIES EN MASSE POUR 100 PARTIES DE PATE : SMA® 20 ET PVP 12.**

a) dans un malaxeur à fort cisaillement, on prépare, comme dans l'exemple précédent, une solution éthanolique à 50 % de SMA 1440-A® ;
b) on ajoute, à 40 parties de cette solution, 12 parties de polyvinylpyrrolidone. On mélange jusqu'à complète homogénéisation ;
c) on ajoute au mélange ainsi obtenu 5 parties d'huile blanche paraffinique ONDINA-32® et 10 parties d'HYDROGRAL-M®. On homogénéise le tout ;
d) on introduit alors dans le malaxeur un mélange comprenant 10 parties de gélatine, 10 parties de pectine, 10 parties de carboxyméthylcellulose sodique, puis 3 parties de THIXOMEN® et enfin une quantité de vanilline correspondant à 0,1 % de la composition finale.
La pâte obtenue a donc la composition suivante :

| | Parties en masse pour 100 parties de pâte |
|---|---|
| SMA 1440-A® | 20 |
| PVP | 12 |
| Gélatine | 10 |
| Pectine | 10 |
| CMC Na | 10 |
| THIXOMEN® | 3 |
| Ethanol | 20 |
| Huile paraffinique ONDINA-32® | 5 |
| HYDROGRAL-M® | 10 |
| Vanilline | 0,1 %. |

**EXEMPLE 3 : PROCEDE DE PREPARATION D'UNE PATE CONFORME A L' INVENTION AVEC EN PARTIES EN MASSE POUR 100 PARTIES DE PATE : SMA® 30 ET PVP 11.**

a) dans un malaxeur, on mélange 30 parties en masse de SMA 1440-A® et 25 parties en masse d'éthanol ;

b) on ajoute à la solution éthanolique de résine ainsi obtenue 11 parties de polyvinylpyrrolidone. On mélange jusqu'à complète homogénéisation ;

c) on ajoute à ce mélange 3 parties d'HYDROGRAL-M® ;

d) on introduit, dans le malaxeur, un mélange comprenant 9 parties de gélatine, 9 parties de pectine, 9 parties de carboxyméthylcellulose et 2 parties d'hydroxypropylcellulose, puis 2 parties de THIXOMEN® et une quantité de vanilline correspondant à 0,1 % de la formulation finale.

La pâte obtenue a donc la composition suivante :

| | Parties en masse pour 100 parties de pâte |
|---|---|
| SMA 1440-A® | 30 |
| PVP | 11 |
| Gélatine | 9 |
| Pectine | 9 |
| CMC Na | 9 |
| Hydroxypropylcellulose | 2 |
| THIXOMEN® | 2 |
| Ethanol | 25 |
| HYDROGRAL-M® | 10 |
| Vanilline | 0,1 %. |

## ETUDE QUALITATIVE DES PATES CONFORMES A L'INVENTION.

Les propriétés des pâtes préparées conformément aux exemples 1, 2 et 3, ci-après désignées pâtes 1, 2 et 3, ont fait l'objet d'une étude comparative avec une pâte largement commercialisée, pour la protection de la peau, dans le domaine des stomies et ci-après désignée pâte de l'état de la technique. Selon le Brevet EP 48 556, cette pâte est constituée d'un mélange comprenant :

. de 25 à 28 % de l'hémi-ester butylique de poly(méthylvinyléther/anhydride maléique) en tant qu'agent filmogène,

. de 35 à 38 % d'hydrocolloïdes (gélatine, pectine, carboxyméthylcellulose sodique),

. de 0,1 à 5 % de triacétate de glycéryle (agent plastifiant),

. de 0,8 à 2 % de Thixin E (agent gélifiant), et

. de 25 à 45 % d'éthanol.

L'étude comparative, entre les propriétés des 3 exemples de pâtes conformes à l'invention et celles de la pâte de l'état de la technique, a porté sur :

1. la consistance de chacune des pâtes,
2. leur facilité à être étalée sur la peau,
3. leur adhésivité instantanée,
4. leur tenue à des tests dits de fluage,
5. leur pouvoir d'absorption des liquides, notamment d'une solution hydrosaline (NaCl 9 g/l),
6. leur degré de gonflement suite à cette absorption.

A - Protocoles opératoires :

1. La qualité d'étalement est appréciée notamment par un test consistant à appliquer une quantité (à peu près reproductible) de pâte sur une surface cutanée humide (dos de la main par exemple), puis à l'étaler à l'aide d'un doigt humidifié. Après évaporation partielle du solvant, on observe la formation ou non d'un film.

2. L'adhésivité instantanée est, elle, appréciée par la plus ou moins grande difficulté éprouvée pour la séparation des doigts, après étalement de la pâte.

3. Les tests de fluage répondent à la nécessité de développer des essais mécaniques capables de chiffrer

les contraintes supportées par les pâtes dans des conditions modélisant au mieux celles de mise en oeuvre effective (poche de recueil, par exemple de stomie, collée sur le corps du patient en station debout et atteignant en fin d'utilisation la charge maximale). Ces essais consistent à déposer sur une surface non tissée, maintenue uniformément humide par une éponge et placée sur le dessus d'une plaque perforée recouvrant un bac à circulation d'eau de température comprise entre 35 et 38°C (modélisation d'une surface cutanée humide), une quantité reproductible de pâte adhésive, puis, après évaporation partielle du solvant, à recouvrir le joint ainsi formé par une bande de taille définie (éprouvette de test) faite d'un non tissé et d'une gomme adhésive reproduisant le support adhésif d'une poche de recueil.

Les éprouvettes sont soumises à des efforts de cisaillement. Leur tenue à ces contraintes est appréciée par la mesure du temps s'écoulant avant qu'elles ne se décollent ou ne s'arrachent.

4. Le comportement des pâtes en présence d'eau a été étudié en observant l'évolution d'un échantillon de pâte au contact d'un volume déterminé de solution hydrosaline.

La quantité d'eau absorbée par un échantillon de pâte est déterminée par un test consistant à déposer et à étaler sur une plaque en verre une quantité de pâte préalablement pesée. On applique sur l'échantillon ainsi préparé un cylindre en verre et la partie de l'échantillon entourant la circonférence du cylindre est éliminée. Le cylindre est alors rempli de solution hydrosaline de telle sorte que celle-ci soit en excès par rapport au volume de l'échantillon. A l'issue d'une période de 24 heures, l'échantillon est pesé, et la quantité de solution absorbée ainsi déterminée.

Le degré de gonflement d'un échantillon de pâte est, lui, apprécié par un test consistant à déposer, à l'intérieur d'une seringue, un volume déterminé de pâte, puis à ajouter une quantité en excès de solution hydrosaline. Une "hydrolyse" de la pâte, liée à l'absorption d'eau par les hydrocolloïdes, se développe à partir de la zone de contact entre l'échantillon de pâte et la solution hydrosaline et se manifeste par une modification de son aspect et notamment de sa couleur. A l'issue d'un temps de 24 heures, on mesure le volume de la fraction "hydrolysée" de pâte. Le rapport entre ce dernier et le volume qu'occupait cette même fraction avant qu'elle ne commence à s'"hydrolyser" permet de définir le degré de gonflement de l'échantillon de pâte.

B - <u>Résultats</u> :

Les résultats de ces essais comparatifs sont exprimés dans le Tableau I ci-après.

Ils montrent que les pâtes préparées conformément à l'invention sont, non seulement homogènes, mais de plus thixotropes, ce qui n'est pas le cas de la pâte de l'état de la technique. Cette dernière présente, en effet, une viscosité qui est constante, qu'elle soit ou non soumise à des contraintes de cisaillement. Si cette viscosité permet de la faire sortir aisément de son conditionnement, elle se révèle être insuffisante lors de son application sur la peau, ce qui se traduit par exemple par des coulées de pâte, notamment dans l'orifice chirurgical. Ces inconvénients sont évités avec les pâtes conformes à l'invention en raison de leur thixotropie.

Les pâtes 1 et 2 présentent une facilité d'étalement comparable à celle de la pâte de l'état de la technique. Seule, la pâte 3 apparaît s'étaler moins facilement, sans doute en raison de l'absence, dans cette composition, de diluant huileux.

L'adhésivité instantanée des pâtes conformes à l'invention est au moins équivalente à celle de la pâte de l'état de la technique. Les pâtes 2 et 3 présentent même une pégosité supérieure due à la présence, dans leurs formulations, d'une résine tackifiante.

Les tests dits de fluage montrent que, dans le cas des pâtes conformes à l'invention, le décollement des éprouvettes sous l'effet des charges de traction est obtenu au delà de 48 heures alors qu'il se produit, dans des conditions expérimentales strictement identiques, aux environs de la 24ème heure pour la pâte de l'état de la technique.

Par ailleurs, si l'on observe une capacité à absorber les liquides comparable chez toutes les pâtes testées (supérieure à 400 % à l'issue d'une période de 24 heures), il apparaît, en revanche, un gonflement des pâtes 1, 2 et 3 inférieur à celui de la pâte de l'état de la technique. Selon les formulations, les pâtes conformes à l'invention présentent un degré de gonflement compris entre 200 et 230 % à la 24ème heure, alors que la pâte de l'état de la technique a subi un gonflement de 275 % dans les mêmes conditions. Or, on sait, à présent, que l'hydratation (et son corollaire, l'"hydrolyse") constitue la première cause du processus de vieillissement et de dégradation des compositions adhésives à usage cutané.

Ces résultats traduisent donc une résistance à l'humidité supérieure des pâtes conformes à l'invention, résistance qui conditionne le maintien de leurs qualités d'adhésivité et d'étanchéité.

TABLEAU I

| | Consistance | Etalement | Adhésivité instantanée | Tenue aux tests de fluage | Absorption d'eau à 24 heures | Gonflement à 24 heures |
|---|---|---|---|---|---|---|
| Pâte 1 | Homogène et Thixotrope | Très bon | Bonne | >48 heures | >400% | <250% |
| Pâte 2 | Homogène et Thixotrope | Très bon | Très bonne | >48 heures | >400% | <250% |
| Pâte 3 | Homogène et Thixotrope | Bon | Très bonne | >48 heures | >400% | <250% |
| Pâte de l'état de la technique | Homogène | Très bon | Bonne | 24 heures | >400% | >250% |

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

**1°)** Pâte adhésive pour la protection de la peau, comprenant au moins un agent filmogène, une charge hydrophile et un solvant alcoolique, caractérisée en ce que l'agent filmogène est choisi parmi des copolymères de styrène et d'anhydride maléique solubles dans l'alcool, la charge hydrophile est une association de polyvinylpyrrolidone et d'au moins un hydrocolloïde soluble et/ou gonflant dans l'eau, et en ce que ladite pâte adhésive contient, en outre, au moins un agent thixotropant.

**2°)** Pâte adhésive selon la revendication 1, caractérisée en ce qu'elle comprend, en outre, au moins l'un des additifs suivants : un diluant huileux et/ou un agent odoriférant et/ou une résine tackifiante.

**3°)** Pâte adhésive selon la revendication 1 ou la revendication 2, caractérisée en ce que l'agent filmogène est choisi parmi les copolymères de styrène et d'anhydride maléique partiellement estérifiés par des alcools et présentant la formule I ci-après :

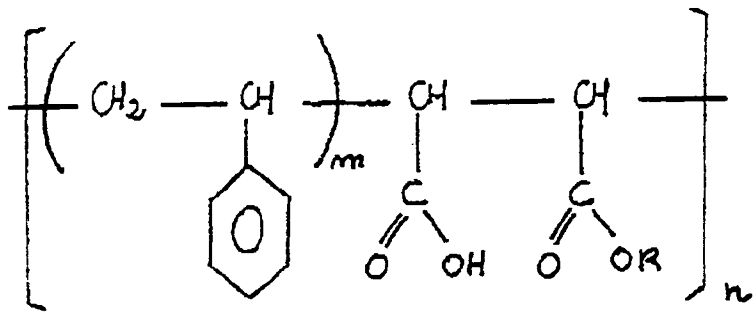

dans laquelle m = 1-3 et n = 6-8.

**4°)** Pâte adhésive selon l'une quelconque des revendications 1 à 3, caractérisée en ce que l'agent filmogène est l'hémi-ester du butylcellosolve du copolymère de styrène et d'anhydride maléique.

**5°)** Pâte adhésive selon l'une quelconque des revendications 1 à 4, caractérisée en ce que le rapport en masses agent filmogène: polyvinylpyrrolidone est compris entre 3,5:1 et 2:1.

**6°)** Pâte adhésive selon l'une quelconque des revendications 1 à 5, caractérisée en ce que le solvant est choisi parmi l'éthanol, le n-propanol, l'isopropanol ou un mélange de ceux-ci.

**7°)** Pâte adhésive selon l'une quelconque des revendications 1 à 6, caractérisée en ce que le ou les hydrocolloïdes solubles et/ou gonflant dans l'eau sont choisis parmi :

- les polysaccharides : carboxyméthylcelluloses (sodiques), hydroxypropylcelluloses, amidons, pectines, dextranomères, gommes naturelles (arabique, de guar, de caroube, de karaya, adragante, xanthane), alginates,...
- les polypeptides : gélatine, caséine, gluten,...,
- les polymères de synthèse hydrosolubles tels que les polyoxyéthylènes ou les polyacrylamides.

**8°)** Pâte adhésive selon la revendication 7, caractérisée en ce qu'elle comprend un mélange d'hydrocolloïdes comprenant de la gélatine, une pectine, une carboxyméthylcellulose sodique et une hydroxypropylcellulose.

**9°)** Pâte adhésive selon l'une quelconque des revendications 1 à 8, caractérisée en ce que l'agent thixotropant est choisi parmi les dérivés solides de l'huile de ricin ou les poudres minérales de la famille des argiles smectites telles que la bentonite et/ou la montmorillonite.

**10°)** Pâte adhésive selon l'une quelconque des revendications 2 à 9, caractérisée en ce que le diluant huileux est choisi parmi les lactates d'acides gras, les adipates ou les huiles blanches paraffiniques de qualité pharmaceutique.

**11°)** Pâte adhésive selon l'une quelconque des revendications 2 à 10, caractérisée en ce que l'agent odoriférant est choisi parmi la vanilline et les essences aromatiques.

**12°)** Pâte adhésive selon l'une quelconque des revendications 2 à 11, caractérisée en ce que la résine tackifiante est choisie parmi les résines terpéniques telles que les esters de collophane ou les polypinènes.

**13°)** Pâte adhésive selon l'une quelconque des revendications 1 à 12, caractérisée en ce qu'elle comprend

en parties en masse :

. 10 à 40 parties d'agent filmogène,
. 5 à 15 parties de polyvinylpyrrolidone,
. 5 à 60 parties d'hydrocolloïdes,
. 1 à 5 parties d'agent thixotropant,
. 10 à 40 parties de solvant alcoolique,
. 0 à 10 parties de diluant huileux,
. 0 à 20 parties de résine tackifiante,
. 0 à 1 partie d'agent odoriférant.

**14°)** Pâte adhésive selon l'une quelconque des revendications 1 à 13, caractérisée en ce qu'elle comprend en parties en masse :

. 20 à 30 parties de l'hémi-ester du butylcellosolve du copolymère de styrène/anhydride maléique,
. 8 à 12 parties de polyvinylpyrrolidone,
. 24 à 50 parties d'hydrocolloïdes :
dont
- 8 à 15 parties de gélatine,
- 8 à 15 parties de pectine,
- 8 à 15 parties de carboxyméthylcellulose sodique,
- 0 à 5 parties d'hydroxypropylcellulose,

. 1 à 3 parties d'agent thixotropant,
. 20 à 25 parties d'éthanol,
. 2 à 10 parties de diluant huileux,
. 0 à 10 parties de résine tackifiante,
. 0,1 partie d'agent odoriférant.

**15°)** Pâte adhésive selon l'une quelconque des revendications 1 à 14, caractérisée en ce qu'elle comprend en parties en masse :

. 25 parties de l'hémi-ester du butylcellosolve du copolymère de styrène/anhydride maléique,
. 10 parties de polyvinylpyrrolidone,
. 37 parties d'hydrocolloïdes :
dont
- 12 parties de gélatine,
- 12 parties de pectine,
- 12 parties de carboxyméthylcellulose sodique,
- 1 partie d'hydroxypropylcellulose,

. 1 partie d'agent thixotropant,
. 25 parties d'éthanol,
. 2 parties de diluant huileux,
. 0,1 partie de vanilline.

**16°)** Procédé de préparation de la pâte adhésive selon l'une quelconque des revendications 1 à 15, caractérisé en ce que :

- dans un malaxeur, on mélange l'agent filmogène et le solvant alcoolique pour obtenir une solution alcoolique dudit agent filmogène ; on ajoute à celle-ci la polyvinylpyrrolidone, puis, le cas échéant, le diluant huileux et/ou la résine tackifiante et on homogénéise ce mélange ;
- on y incorpore le ou les hydrocolloïdes, l'agent thixotropant, et, le cas échéant, l'agent odoriférant et l'ensemble est malaxé jusqu'à l'obtention d'une pâte totalement homogène.

**17°)** Compositions pharmaceutiques pour la protection de la peau, caractérisées en ce qu'elles comprennent en tant que constituant actif une pâte selon l'une quelconque des revendications 1 à 15.

**18°)** Compositions pharmaceutiques, caractérisées en ce qu'elles contiennent en combinaison une pâte selon l'une quelconque des revendications 1 à 15 et d'autres substances à activité thérapeutique.

**19°)** Compositions pharmaceutiques à diffusion contrôlée, caractérisées en ce qu'elles comprennent en tant que véhicule de diffusion, la pâte selon l'une quelconque des revendications 1 à 15.

Office européen des brevets

# RAPPORT DE RECHERCHE EUROPEENNE

Numero de la demande
EP 93 40 1733

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| | Aucun document pertinent relevé<br>----- | | A61L25/00 |
| | | | DOMAINES TECHNIQUES RECHERCHES (Int.Cl.5) |
| | | | A61L |

Le présent rapport a été établi pour toutes les revendications

| Lieu de la recherche | Date d'achèvement de la recherche | Examinateur |
|---|---|---|
| LA HAYE | 29 Octobre 1993 | PELTRE, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)